# FASCICULE DE BREVET EUROPEEN

(11) **EP 2 981 217 B1**
(45) Date de publication et mention de la délivrance du brevet: **15.02.2017**
(21) Numéro de dépôt: 14718630.8
(22) Date de dépôt: 24.03.2014
(51) Int. Cl.: A61B 17/122, A61B 17/11

(54) **SYSTÈME POUR ABOUTER DEUX VAISSEAUX SANGUINS**
SYSTEM ZUM VERBINDEN ZWEIER BLUTGEFÄSSE
SYSTEM FOR JOINING TWO BLOOD VESSELS

(30) Priorité: 02.04.2013 FR 1300751
(43) Date de publication de la demande: 10.02.2016
(73) Titulaire: Renard, Xavier, 91430 Vauhallan (FR)
(72) Inventeur: MASONI, Bruno, 52700 Ozieres (FR)
(74) Mandataire: Flavenot, Bernard
(86) Numéro de dépôt international: PCT/FR2014/000060
(87) Numéro de publication internationale: WO 2014/162067

(56) Documents cités:
- EP-A1- 0 105 414
- WO-A2-2004/073487
- US-A- 5 103 839

## Description

La présente invention concerne les systèmes pour abouter deux vaisseaux sanguins au moyen de pinces vasculaires qui sont plus généralement connues des hommes du métier sous la terminologie anglo-saxonne "clamp" vasculaire.

De telles pinces sont couramment utilisées en chirurgie, notamment de la main et du pied, lorsqu'il est nécessaire de manipuler, tenir, abouter, etc., par exemple pour les suturer, des petits vaisseaux sanguins pendant une opération chirurgicale.

De telles pinces sont déjà connues, comme celle décrite et illustrée dans le EP 0 105 414, représentant l'état de l'art le plus proche de la présente invention, et aussi par exemple dans les US 5103839 et WO 2004/073487.

Cette pince comporte essentiellement deux pattes sensiblement identiques, des moyens pour monter ces deux pattes en rotation l'une par rapport à l'autre autour d'un axe de rotation situé sensiblement à proximité de la partie médiane des deux pattes, délimitant ainsi, sur chaque patte, deux première et seconde portions situées de part et d'autre d'un plan contenant cet axe de rotation et sensiblement perpendiculaire au plan général d'au moins l'une des pattes, et des moyens pour appliquer, sur les premières portions de pattes, respectivement deux forces antagonistes élastiques de façon que les deux extrémités libres des premières portions de pattes aient tendance à s'éloigner l'une de l'autre et que corrélativement les extrémités libres des deux secondes portions de pattes aient tendance à venir au contact l'une de l'autre.

Une réalisation comme décrite ci-dessus donne les résultats attendus avec une relative satisfaction. Cependant, elle présente l'inconvénient majeur de conférer au système un coût de revient qui s'avère relativement élevé compte tenu du fait que ce type de pince vasculaire est un dispositif à usage unique et qu'il gêne parfois le praticien pendant l'opération de suture.

Aussi, la présente invention a-t-elle pour but de réaliser un système pour abouter deux vaisseaux sanguins qui tente de pallier au moins en partie les inconvénients mentionnés ci-dessus des systèmes de l'art antérieur tels que décrits ci-dessus, et qui en plus puisse répondre à certaines demandes des chirurgiens, par exemple en leur permettant de choisir ou modifier la pression exercée par la pince sur le vaisseau qu'elle doit maintenir, d'appliquer une force de pression modulable selon une direction définie par l'axe longitudinal des faisceaux sanguins afin d'assurer leur suture.

Mais surtout, la présente invention a aussi pour but de proposer un système qui facilite le travail opératoire des praticiens pour réaliser la suture entre deux vaisseaux sanguins.

Plus précisément, la présente invention a pour objet un système pour abouter deux vaisseaux sanguins, caractérisé par le fait qu'il comporte au moins deux pinces, chaque pince comportant : deux pattes sensiblement identiques, des moyens pour monter les deux dites pattes en rotation l'une par rapport à l'autre autour d'un axe de rotation, ledit axe de rotation étant situé sensiblement à proximité des parties médianes respectivement des deux pattes en délimitant de ce fait, sur chacune des pattes, une première portion et une seconde portion situées de part et d'autre d'un premier plan contenant ledit axe de rotation et sensiblement perpendiculaire au plan général d'au moins l'une des deux pattes ; des moyens pour appliquer, sur les deux premières portions de pattes, respectivement deux forces élastiques antagonistes de façon que les deux extrémités libres de ces premières portions de pattes aient tendance à s'éloigner l'une de l'autre et que, corrélativement, les extrémités libres des deux secondes portions de pattes aient tendance à venir au contact l'une de l'autre ; et une percée traversante réalisée dans l'une des deux pattes selon un axe sensiblement parallèle au dit axe de rotation, ladite percée traversante étant cylindrique à section transversale polygonale ; ledit système comportant en outre une tige d'axe longitudinal et ayant une section transversale complémentaire de celle de la percée traversante, ladite tige étant enfichée dans les deux percées traversantes respectivement des deux pinces, caractérisé par le fait qu'une portion dé ladite tige comprise entre les deux pinces possède, suivant deux directions orthogonales entre elles dont l'une est perpendiculaire au premier plan, deux moments d'inertie de valeurs différentes, le moment d'inertie ayant la valeur la plus faible étant celui défini sensiblement selon la direction qui est perpendiculaire au premier plan.

D'autres caractéristiques et avantages de la présente invention apparaîtront au cours de la description suivante donnée en regard des dessins annexés à titre illustratif mais nullement limitatif, dans lesquels :
La figure 1 est un schéma de principe d'une pince vasculaire entrant dans la constitution du système selon l'invention pour abouter au moins deux vaisseaux sanguins,
Les figures 2, 3 et 4 représentent l'un des éléments constitutifs de la pince vasculaire selon la figure 1, dans des vues respectivement de dessous, de côté et en perspective,
Les figures 5, 6 et 7 représentent un autre des éléments constitutifs de la pince vasculaire selon la figure 1, dans des vues respectivement de dessous, de côté et en perspective,
La figure 8 est une vue en perspective et en éclaté d'un mode de réalisation de la pince vasculaire selon les figures 1 à 7,
La figure 9 est une vue de dessus d'un élément permettant de réaliser l'un des éléments constitutifs de la pince vasculaire selon la figure 8, et
Les figures 10, 11 et 12 représentent, prises en combinaison, deux modes de réalisation d'un système selon l'invention pour abouter deux vaisseaux sanguins, le système comportant deux pinces vasculaires selon les figures 1 à 8, reliées par une tige, la figure 10 étant une vue de dessus, la figure 11 étant une vue en coupe référencée *XI-XI* sur la figure 10, la figure 12 représentant, en vue de dessus, un mode de réalisation possible d'un des éléments illustrés sur la figure 10.

Il est tout d'abord précisé que, dans la présente description, si l'adverbe "sensiblement" est associé à un qualificatif d'un moyen donné, ce qualificatif doit être compris au sens strict ou approché.

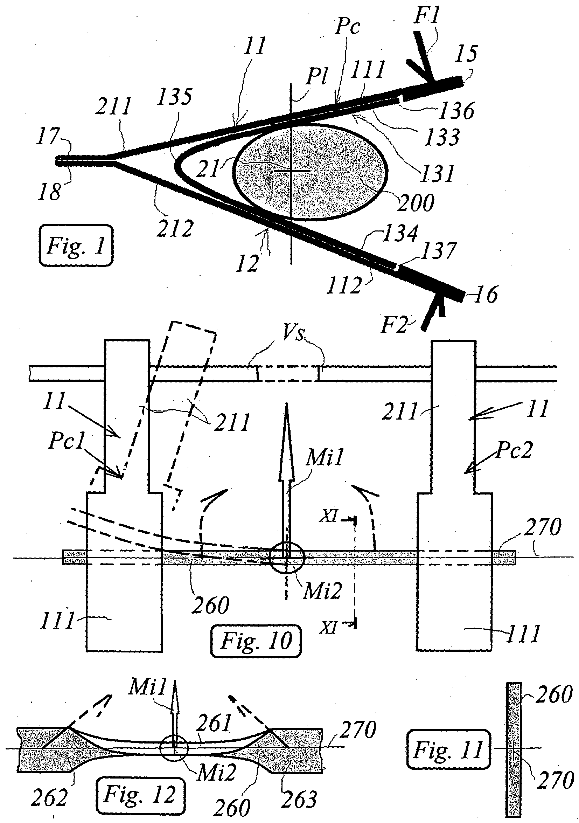

De plus, l'échelle selon laquelle certains éléments sont représentés sur les figures n'est pas homogène. Pour certains détails de ces éléments, l'échelle utilisée, par exemple pour la figure 10, n'est pas la même selon deux directions non confondues. Ce type de représentation a été adopté pour bien faire ressortir, par exagération, les caractéristiques structurelles des différents éléments.

En référence aux figures annexées, la présente invention concerne un système pour abouter au moins deux vaisseaux sanguins Vs, comprenant deux pinces vasculaires Pc dénommées par les hommes du métier sous le terme de "clamp", pour par exemple suturer ces deux vaisseaux sanguins.

Chaque pince vasculaire Pc comporte deux pattes 11, 12 sensiblement identiques et des moyens 20 pour monter ces deux pattes en rotation l'une par rapport à l'autre autour d'un axe de rotation 21 situé sensiblement à proximité des parties médianes 13, 14 respectivement des deux pattes.

Ces moyens de rotation 20 peuvent être par exemple du même type que ceux décrits et illustrés dans le document antérieur mentionné au préambule de la présente description.

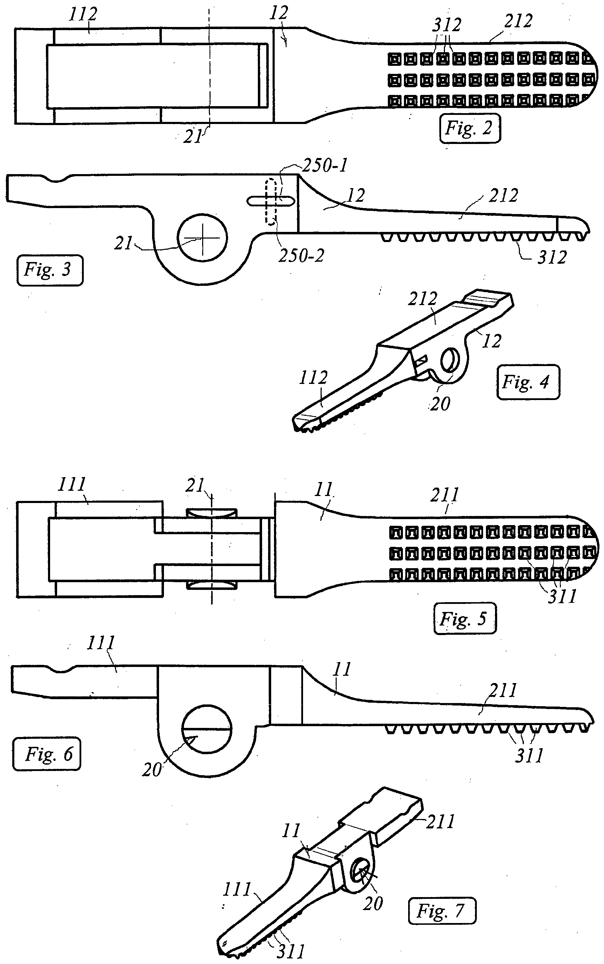

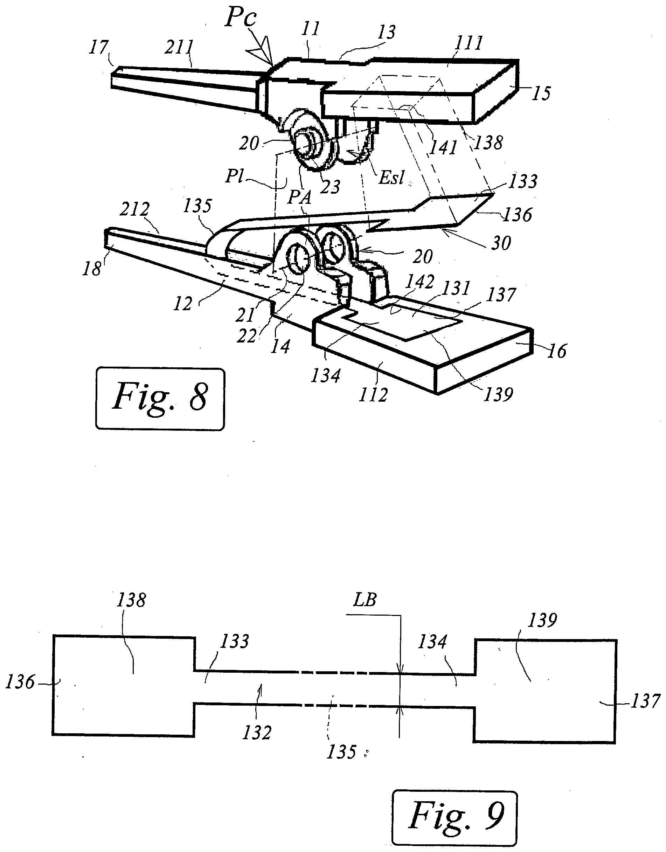

Comme illustré sur les figures 2 à 8, ces moyens de rotation 20 sont avantageusement constitués par au moins un système de rotation PA comportant un palier 22 et un arbre 23 complémentaire du palier, le palier étant constitué d'une bague cylindrique sensiblement de révolution autour de l'axe de rotation 21 de façon que l'arbre 23 soit enclipsable dans la bague. Mais, de façon encore plus préférentielle, ces moyens 20 comportent deux systèmes de rotation PA sensiblement identiques, éloignés l'un de l'autre pour dégager, entre eux, un espace libre Esl.

De ce fait, ces moyens de rotation 20 délimitent, sur chacune des deux pattes 11, 12, une première portion 111, 112 et une seconde portion 211, 212 situées de part et d'autre d'un plan PI contenant l'axe de rotation 21 et sensiblement perpendiculaire au plan général d'au moins l'une des deux pattes.

La pince vasculaire Pc comporte en outre des moyens 30 pour appliquer, sur les deux premières portions de pattes 111, 112, respectivement deux forces élastiques antagonistes de façon que les deux extrémités libres 15, 16 de ces premières portions de pattes aient tendance à s'éloigner l'une de l'autre et que, corrélativement, les extrémités libres 17, 18 des deux secondes portions de pattes 211, 212 aient tendance à venir au contact l'une de l'autre.

Ces moyens 30 pour appliquer, sur les deux premières portions de pattes 111, 112, deux forces élastiques antagonistes sont constitués d'une pièce sensiblement en U 131 formée dans une plaque 132 en un matériau élastique repliée de façon qu'elle comporte deux branches 133, 134, une extrémité de l'une des deux branches étant reliée à une extrémité de l'autre branche par une demi-boucle 135 ouverte sur au moins quatre-vingt-dix degrés d'angle, la pièce en U étant fichée entre les deux pattes 11, 12.

De préférence, pour obtenir un équilibre statique mais aussi dynamique de la pince, la demi-boucle ouverte 135 est positionnée sensiblement à proximité de l'axe de rotation 21.

Au sens de la présente description, par "plaque", il est compris un élément oblong et relativement plat dont la largeur est nettement supérieure à l'épaisseur.

La pince vasculaire Pc comporte en outre des moyens pour lier en butée les deux extrémités libres 136, 137 respectivement des deux branches 133, 134 de la pièce en U respectivement avec les faces en regard des deux premières portions de pattes 111, 112 de façon que cette pièce en U n'ait pas tendance, par détente naturelle, à glisser en dehors de l'espace en V défini entre des deux pattes 11, 12, figure 1.

Ces moyens pour lier en butée les deux extrémités libres 136, 137 respectivement des deux branches 133, 134 respectivement avec les faces en regard des deux premières portions de pattes 111, 112 sont essentiellement constitués par des logements en creux 141, 142 réalisés dans les faces en regard de ces premières portions de pattes 111, 112 et dans lesquels viennent se loger respectivement les extrémités libres 136, 137 des deux branches 133, 134 de la pièce en U.

Selon une réalisation particulièrement avantageuse, ces extrémités libres 136, 137 des deux branches 133, 134 de la pièce en U sont conformées en parties élargies 138, 139 par rapport au reste de la plaque 132, et les logements ont une forme complémentaire de ces parties élargies de façon que ces dernières viennent s'encastrer de façon congruente respectivement dans les logements 141, 142.

Dans le mode de réalisation décrit ci-dessus, les deux branches 133, 134 de la pièce en U 131 viennent se positionner dans l'espace Esl défini ci-avant et, selon une caractéristique de l'invention, au moins l'une de ces deux branches 133, 134, avantageusement les deux, a une largeur LB sensiblement égale à celle de cet espace libre Esl.

Ainsi, quand les deux ensembles « palier 22 et arbre 23 » respectivement des deux systèmes de rotation PA sont assemblés, les branches empêchent que les paliers 22 et les arbres 23 ne se dissocient, du fait de la largeur de ces branches comme défini ci-dessus.

Selon une réalisation préférentielle, figures 3 à 7, chacune des faces des secondes portions de pattes 211, 212 qui sont en regard l'une de l'autre comporte, en saillie, une pluralité d'ergots 311, 312, ces ergots étant disposés sur ces faces de façon que les ergots situés sur la face de l'une des secondes portions de pattes 211, 212 soient disposés en quinconce par rapport aux ergots situés sur la face de l'autre seconde portion de patte 212, 211 quand ces secondes portions de pattes viennent sensiblement au contact l'une de l'autre sous l'action des deux forces élastiques antagonistes appliquées sur les premières portions de pattes 111, 112.

Selon une autre caractéristique possible de l'invention, le système peut comporter en outre au moins un bloc d'élastomère 200 élastiquement déformable en pression, ce bloc ayant des dimensions lui permettant d'être inséré en force, par déformation, entre les deux premières portions de pattes 111,112.

En outre, ce bloc 200 est avantageusement situé entre les deux branches 133, 134 de la pièce en U 131, et même à proximité, sinon au contact, de la demi-boucle ouverte 135.

De préférence, pour faciliter son utilisation, le bloc 200 présente une forme choisie entre les deux formes suivantes : un cylindre à section transversale sensiblement elliptique, figure 1, dont les axes focaux sont sensiblement parallèles à l'axe de rotation 21 ; un tronc de pyramide inscrit dans un dièdre dont l'arête au sommet est sensiblement parallèle à l'axe de rotation 21 et agencé de façon que les deux faces définissant le dièdre soient au contact respectivement des faces en regard des deux branches 133, 134 de la pièce en U 131.

Cette réalisation est avantageuse car elle permet au Praticien d'ajuster à son choix la valeur de la pression qui peut être obtenue au niveau des extrémités libres 17, 18 des deux secondes portions de pattes 211, 212 afin d'obtenir l'intensité souhaitée pour la force de pincement appliquée sur les vaisseaux sanguins Vs, soit en n'utilisant pas un bloc 200 tel que décrit ci-dessus, soit en insérant un tel bloc entre les deux branches 133, 134 de la pièce en U 131 s'il veut augmenter la pression exercée sur les vaisseaux sanguins. Selon un avantage de ce mode de réalisation du système selon l'invention, le Praticien pourra ainsi choisir, par exemple au moyen d'abaques prédéterminés, un bloc 200 dans un ensemble de blocs de différentes formes mis à sa disposition, afin d'obtenir une pression souhaitée sur les vaisseaux sanguins qui doivent être aboutés.

De façon avantageuse, pour faciliter l'utilisation, par le praticien, du système selon l'invention lors d'une intervention chirurgicale, l'une des deux pattes 11, 12 de chaque pince Pc1, Pc2 comporte une percée traversante 250 cylindrique et à section transversale polygonale, réalisée selon un axe sensiblement parallèle à l'axe de rotation 21, et le système comporte en outre une tige 260 d'axe longitudinal 270 et ayant une section transversale complémentaire de celle de la percée traversante, la tige étant enfichée dans les deux percées traversantes 250 respectivement des deux pinces avec un degré de friction qui ne facilite pas facilement le coulissement des pinces le long de cette tige.

Selon une caractéristique de l'invention, au moins une portion de la tige 260 comprise entre les deux pinces Pc1, Pc2, possède, suivant deux directions orthogonales entre elles dont l'une est perpendiculaire au premier plan PI, deux moments d'inertie Mi1, Mi2 de valeurs différentes, le moment d'inertie Mi1 ayant la valeur la plus faible étant celui défini sensiblement selon la direction qui est perpendiculaire au premier plan PI, l'autre moment d'inertie Mi2 étant donc celui défini selon une direction sensiblement parallèle à ce plan Pl.

Selon une réalisation possible, figures 10 et 11, la tige 260 est constituée d'une plaque plane définie sur toute sa longueur dans un même plan et ayant une épaisseur inférieure à sa largeur, figure 11, et la section transversale de la percée traversante 250-1 est sensiblement rectangulaire, cette percée traversante présentant une profondeur définie selon une direction parallèle à l'axe de rotation 21, une largeur et une longueur définies respectivement selon deux directions perpendiculaires à cet axe de rotation 21. Dans ce mode de réalisation de la tige 260, la longueur de la percée traversante 250-1 est sensiblement parallèle au premier plan PI, comme représenté en traits interrompus sur la figure 3.

Selon une autre réalisation possible, figures 10 et 12, la tige 260 est constituée d'une plaque, figure 12, comportant une première partie médiane 261 définie dans un plan et située entre deux secondes parties 262, 263 définies dans un plan sensiblement perpendiculaire au plan de la première partie. La section transversale de la percée traversante 250-2 est, comme celle de la percée traversante 250-1, sensiblement rectangulaire, mais sa longueur est sensiblement perpendiculaire au premier plan PI, comme représenté en trait continu sur la figure 3, les deux secondes parties 262, 263 de la tige étant alors respectivement enfichées dans les deux percées traversantes 250-2 respectivement des deux pinces.

Selon une caractéristique de l'invention, dans le but de cette invention, la tige 260 est réalisée en un matériau dont la déformation élastique sous des efforts de torsion est négligeable pour obtenir une déformation plastique prédominante, par exemple en acier inoxydable ou analogue.

En complément des explications données ci-avant, le système selon l'invention pour abouter au moins deux vaisseaux sanguins Vs comportant au moins deux pinces Pc1, Pc2, s'utilise de la façon suivante :

Initialement, au repos, les deux secondes portions de pattes 211, 212 des pinces Pc sont au contact l'une de l'autre par leurs extrémités libres 17, 18, alors que les extrémités libres 15, 16 des deux premières portions de pattes 111, 112 sont éloignées l'une de l'autre au maximum.

Pour pincer un des deux vaisseaux sanguins, le Praticien appuie en opposition F1, F2 sur les extrémités libres 15, 16 des premières portions de pattes pour les rapprocher l'une de l'autre à l'encontre de la force élastique exercée par la pièce en U, et le bloc d'élastomère 200, s'il est présent, qui se comprime encore plus. En conséquence, les extrémités libres 17, 18 des secondes portions de pattes s'écartent l'une de l'autre et le Praticien peut les placer de part et d'autre du vaisseau sanguin. Lorsqu'il relâche les extrémités 15, 16, les deux extrémités 17, 18 se rapprochent l'une de l'autre et pincent le vaisseau sanguin.

Pour abouter deux vaisseaux sanguins Vs, il est nécessaire d'utiliser au moins deux pinces vasculaires en parallèle en les couplant, comme décrit auparavant en regard de la figure 10, sur une tige 260.

Dans ce cas et selon sa structure illustrée sur la figure 10, le système s'utilise de la façon suivante:

Les deux pinces Pc1, Pc2 sont positionnées sur la tige 260 comme décrit ci-avant et à une distance relativement plus importante l'une de l'autre qu'avec les solutions de l'art antérieur, puis pincées respectivement sur les deux vaisseaux Vs, figure 10, dont les extrémités sectionnées sont par exemple à suturer, de façon que leurs extrémités séparées soient à proximité l'une de l'autre.

Puis la tige 260 est pliée autour d'un axe parallèle au plan PI, soit manuellement soit au moyen d'un ancillaire adapté, ce qui est relativement facile du fait de la structure de la tige selon l'invention, notamment le fait que le moment d'inertie Mi1 qui est le plus faible est défini suivant une direction sensiblement perpendiculaire à ce plan Pl.

Ce pliage s'effectue jusqu'à ce que les deux extrémités sectionnées des deux vaisseaux sanguins soient au contact l'une de l'autre avec une certaine pression.

Selon le processus décrit ci-dessus, les extrémités libres des secondes portions de pattes des deux pinces sont plus proches les unes des autres que les extrémités libres des premières portions de pattes.

Ainsi, les deux pinces se trouvent être en position oblique l'une par rapport à l'autre, comme illustré en traits interrompus pour la pince Pc1 et en traits pleins pour la pince Pc2 sur la figure 10. Cette disposition permet de définir entre les pinces un espace libre beaucoup plus important qu'avec les solutions de l'art antérieur, bien que les extrémités libres des premières portions de pattes puissent être amenées aussi proches l'une de l'autre qu'avec ces solutions antérieures, permettant ainsi au Praticien d'avoir une accessibilité aux extrémités des deux vaisseaux à suturer plus grande que lorsque les deux pinces sont parallèles entre elles. Le Praticien peut alors, avec plus d'aisance et de sécurité, suturer les deux extrémités de vaisseaux.

Il est cependant possible d'utiliser la tige 260 en pliant sa partie médiane 261 sur environ trois-cent-soixante degrés, de façon qu'elle fasse une boucle fermée et que les deux secondes parties 262, 263 soient en dehors de cette boucle et forment entre elles par exemple un angle droit. Sur ces deux parties, seront alors positionnées les deux pinces Pc. Cette configuration est très avantageuse, par exemple dans le cas de branchements vasculaires terminaux latéraux, notamment de façon verticale, par exemple pour permettre à deux Praticiens d'opérer ensemble.

La description faite ci-dessus met clairement en évidence les avantages présentés par le système selon l'invention pour abouter deux vaisseaux sanguins par rapport aux systèmes de l'art antérieur et montre que les buts recherchés avec la présente invention sont atteints.

## Revendications

1. Système pour abouter deux vaisseaux sanguins (Vs), comportant au moins deux pinces (Pc), chaque pince comportant : deux pattes (11, 12) sensiblement identiques, des moyens (20) pour monter les deux dites pattes (11, 12) en rotation l'une par rapport à l'autre autour d'un axe de rotation (21), ledit axe de rotation (21) étant situé sensiblement à proximité des parties médianes (13, 14) respectivement des deux pattes en délimitant de ce fait, sur chacune des pattes, une première portion (111, 112) et une seconde portion (211, 212) situées de part et d'autre d'un premier plan (PI) contenant ledit axe de rotation (21) et sensiblement perpendiculaire au plan général d'au moins l'une des deux pattes ; des moyens (30) pour appliquer, sur les deux premières portions de pattes (111, 112), respectivement deux forces élastiques antagonistes de façon que les deux extrémités libres (15, 16) de ces premières portions de pattes (111, 112) aient tendance à s'éloigner l'une de l'autre et que, corrélativement, les extrémités libres (17, 18) des deux secondes portions de pattes (211, 212) aient tendance à venir au contact l'une de l'autre ; et une percée traversante (250) réalisée dans l'une des deux pattes selon un axe sensiblement parallèle au dit axe de rotation (21), ladite percée traversante étant cylindrique à section transversale polygonale ; ledit système comportant en outre une tige (260) d'axe longitudinal (270) et ayant une section transversale complémentaire de celle de la percée traversante, ladite tige étant enfichée dans les deux percées traversantes (250) respectivement des deux pinces, une portion de ladite tige comprise entre les deux pinces possède, suivant deux directions orthogonales entre elles dont l'une est perpendiculaire au premier plan (PI), deux moments d'inertie de valeurs différentes,
**caractérisé par le fait que** le moment d'inertie ayant la valeur la plus faible étant celui sensiblement défini selon la direction qui est perpendiculaire au dit premier plan (Pl).

2. Système selon la revendication 1, **caractérisé par le fait que** ladite tige (260) est constituée d'une plaque plane définie sur toute sa longueur dans un même plan et ayant une épaisseur inférieure à sa largeur, et que la section transversale de la percée traversante (250-1) est sensiblement rectangulaire, ladite percée traversante présentant une profondeur définie selon une direction parallèle au dit axe de rotation (21), une largeur et une longueur définies respectivement selon deux directions perpendiculaires au dit axe de rotation (21), la longueur étant sensiblement parallèle au dit premier plan (PI).

3. Système selon la revendication 1, **caractérisé par le fait que** ladite tige (260) est constituée d'une plaque comportant une première partie médiane (261) définie dans un plan et située entre deux secondes parties (262, 263) définies dans un plan sensiblement perpendiculaire au plan de la premier partie, et que la section transversale de la percée traversante (250-2) est sensiblement rectangulaire, ladite percée traversante présentant une profondeur définie selon une direction parallèle au dit axe de rotation (21), une largeur et une longueur définies respectivement selon deux directions perpendiculaires au dit axe de rotation (21), la longueur étant sensiblement perpendiculaire au dit premier plan (Pl), les deux secondes parties (262, 263) de la tige étant respectivement enfichées dans les deux percées traversantes (250-2) respectivement des deux pinces.

4. Système selon l'une des revendications 1 à 3,
**caractérisé par le fait que** lesdits moyens (30) pour appliquer, sur les deux premières portions de pattes (111, 112), deux forces élastiques antagonistes sont constitués par une pièce sensiblement en U (131) formée dans une plaque (132) en un matériau élastique repliée de façon qu'elle comporte deux branches (133, 134), une extrémité de l'une des deux branches étant reliée à une extrémité de l'autre branche par une demi-boucle (135) ouverte sur au moins cent-quatre-vingts degrés d'angle, ladite pièce en U étant fichée entre les deux pattes (11, 12), et que ladite pince comporte en outre des moyens pour lier en butée les deux extrémités libres (136, 137) respectivement des deux branches (133, 134) respectivement avec les faces en regard des deux premières portions de pattes (111, 112).

5. Système selon la revendication 4,
**caractérisé par le fait que** les moyens pour lier en butée les deux extrémités libres (136, 137) respectivement des deux branches (133, 134) respectivement avec les faces en regard des deux premières portions de pattes (111, 112) sont constitués par des logements en creux (141, 142) réalisés dans les faces en regard de ces premières portions de pattes (111, 112) dans lesquels viennent se loger respectivement les extrémités libres (136, 137) des deux branches (133, 134) de ladite pièce en U.

6. Système selon l'une des revendications précédentes, **caractérisé par le fait que** chacune des faces des secondes portions de pattes (211, 212) qui sont en regard l'une de l'autre comporte, en saillie, une pluralité d'ergots (311, 312), lesdits ergots étant disposés sur lesdites faces de façon que les ergots situés sur la face de l'une des secondes portions de pattes (211, 212) soient disposés en quinconce par rapport aux ergots situés sur la face de l'autre seconde portion de patte (212, 211) quand ces secondes portions de pattes viennent sensiblement au contact l'une de l'autre sous l'action des deux forces élastiques antagonistes appliquées sur les premières portions de pattes (111, 112).

7. Système selon l'une des revendications précédentes, **caractérisée par le fait que** lesdits moyens (20) pour monter lesdites branches (11, 12) en rotation l'une par rapport à l'autre autour du dit axe de rotation (21) sont constitués par au moins un,système de rotation (PA) comportant un palier (22) et un arbre (23) complémentaire du dit palier, ledit palier étant constitué par une bague cylindrique sensiblement de révolution autour du dit axe de rotation (21) de façon que ledit arbre (23) soit enclipsable dans ladite bague.

8. Système selon la revendication 7,
**caractérisée par le fait qu'**il comporte deux systèmes de rotation (PA) sensiblement identiques, les deux dits systèmes de rotation étant éloignés l'un de l'autre pour dégager, entre eux, un espace libre (Esl) dans lequel viennent se positionner les deux dites deux branches (133, 134).

9. Système selon la revendication 8 quand elle dépend de la revendication 5, **caractérisé par le fait qu'**au moins l'une des deux branches (133, 134) de ladite pièce sensiblement en U (131) a une largeur (LB) sensiblement égale à celle du dit espace libre (Esl).

10. Système selon l'une des revendications précédentes quand elle dépend de la revendication 3, **caractérisé par le fait que** ladite partie médiane (261) de la tige (260) est pliée de façon qu'elle forme une boucle fermée et que les deux dites secondes parties (262, 263) soient situées en dehors de ladite boucle.

## Patentansprüche

1. System zum Zusammenfügen von zwei Blutgefäßen (Vs), das mindestens zwei Zangen (Pc) umfasst, wobei jede Zange umfasst: zwei im Wesentlichen gleiche Greifbacken (11, 12), Mittel (20) zum Zusammensetzen der zwei Greifbacken (11, 12) in einer Weise, dass sie relativ zueinander um eine Drehachse (21) drehbar sind, wobei sich die Drehachse (21) im Wesentlichen in der Nähe von Mittelteilen (13, 14) der beiden jeweiligen Greifbacken befindet und dadurch auf jeder der Greifbacken einen ersten Abschnitt (111, 112) und einen zweiten Abschnitt (211, 212) begrenzt, die beiderseits einer ersten Ebene (PI), die die Drehachse (21) enthält, und im Wesentlichen senkrecht zu der Grundebene zumindest einer der zwei Greifbacken liegen; Mittel (30), um auf die beiden ersten Greifbackenabschnitte (111, 112) jeweils zwei einander entgegenwirkende elastische Kräfte auszuüben, derart, dass die zwei freien Enden (15, 16) dieser ersten Greifbackenabschnitte (111, 112) bestrebt sind, sich voneinander zu entfernen, und dass auf korrelierte Weise die freien Enden (17, 18) der zwei zweiten Greifbackenabschnitte (211, 212) bestrebt sind, einander zu berühren; und ein Durchgangsloch (250), das in einer der zwei Greifbacken längs einer Achse ausgebildet ist, die zu der Drehachse (21) im Wesentlichen parallel ist, wobei das Durchgangsloch zylindrisch ist und einen polygonalen Querschnitt besitzt; wobei das System außerdem einen Stift (260) mit Längsachse (270) umfasst, der einen Querschnitt besitzt, der zu jenem des Durchgangslochs komplementär ist, wobei der Stift in die zwei jeweiligen Durchgangslöcher (250) der zwei Zangen eingesteckt ist, wobei ein Abschnitt des Stifts, der zwischen den zwei Zangen eingeschlossen ist, in zwei zueinander senkrechten Richtungen, wovon eine zu der ersten Ebene (PI) senkrecht ist, zwei Trägheitsmomente mit unterschiedlichen Werten besitzt,
**dadurch gekennzeichnet, dass**
das Trägheitsmoment, das den kleinsten Wert hat, jenes ist, das im Wesentlichen längs der Richtung definiert ist, die zu der ersten Ebene (PI) senkrecht ist.

2. System nach Anspruch 1, **dadurch gekennzeichnet, dass** der Stift (260) aus einer ebenen Platte gebildet ist, die auf ihrer gesamten Länge in derselben Ebene definiert ist und eine Dicke besitzt, die geringer als ihre Breite ist,
und dass der Querschnitt des Durchgangslochs (250-1) im Wesentlichen rechteckig ist, wobei das Durchgangsloch eine Tiefe, die entlang einer Richtung parallel zu der Drehachse (21) definiert ist, eine Breite und eine Länge, die jeweils entlang zwei zu der Drehachse (21) senkrechten Richtungen definiert sind, aufweist, wobei die Länge zu der ersten Ebene (PI) im Wesentlichen parallel ist.

3. System nach Anspruch 1, **dadurch gekennzeichnet, dass** der Stift (260) aus einer Platte gebildet ist, die einen ersten Mittelabschnitt (261) aufweist, der in einer Ebene definiert ist und zwischen zwei zweiten Teilen (262, 263) liegt, die in einer zu der Ebene des ersten Teils im Wesentlichen senkrechten Ebene definiert sind, und dass der Querschnitt des Durchgangslochs (250-2) im Wesentlichen rechteckig ist, wobei das Durchgangsloch eine Tiefe, die entlang einer Richtung parallel zu der Drehachse (21) definiert ist, eine Breite und eine Länge, die jeweils entlang zweier zu der Drehachse (21) senkrechten Richtungen definiert sind, aufweist, wobei die Länge zu der ersten Ebene (PI) im Wesentlichen senkrecht ist, wobei die zwei zweiten Teile (262, 263) des Stifts in die zwei jeweiligen Durchgangslöcher (250-2) der zwei jeweiligen Zangen eingesteckt ist.

4. System nach einem der Ansprüche 1 bis 3, **dadurch gekennzeichnet, dass** die Mittel (30) zum Ausüben von zwei einander entgegenwirkenden elastischen Kräften auf die beiden ersten Greifbackenabschnitte (111, 112) durch ein im Wesentlichen U-förmiges Teil (131) gebildet sind, das in einer Platte (132) aus elastischem Material, die so gebogen ist, dass sie zwei Schenkel (133, 134) aufweist, ausgebildet ist, wobei ein Ende eines der zwei Schenkel mit einem Ende des anderen Schenkels durch eine Halbschleife (135), die über einen Winkel von mindestens hundertachtzig Grad offen ist, verbunden ist, wobei das U-förmige Teil zwischen die zwei Greifbacken (11, 12) eingesteckt ist, und dass die Zange außerdem Mittel umfasst, um die beiden freien Enden (136, 137) der zwei jeweiligen Schenkel (133, 134) mit den jeweiligen Flächen gegenüber den zwei ersten Greifbackenabschnitten (111, 112) anschlagend zu verbinden.

5. System nach Anspruch 4, **dadurch gekennzeichnet, dass** die Mittel zum anschlagenden Verbinden der zwei freien Enden (136, 137) der zwei jeweiligen Schenkel (133, 134) mit den jeweiligen Flächen gegenüber den zwei ersten Greifbackenabschnitten (111, 112) durch hohle Aufnahmeräume (141, 142) gebildet sind, die in den gegenüberliegenden Flächen dieser ersten Greifbackenabschnitte (111, 112) ausgebildet sind und in denen die jeweiligen freien Enden (136, 137) der zwei Schenkel (133, 134) des U-förmigen Teils aufgenommen werden.

6. System nach einem der vorhergehenden Ansprüche, **dadurch gekennzeichnet, dass** jede der Flächen der zweiten Greifbackenabschnitte (211, 212), die sich einander gegenüber befinden, mehrere vorstehende Noppen (311, 312) aufweist, wobei die Noppen an den Flächen in einer Weise angeordnet sind, dass die Noppen, die auf der Fläche eines der zweiten Greifbackenabschnitte (211, 212) liegen, zu den Noppen, die auf der Fläche des anderen zweiten Greifbackenabschnitts (211, 212) liegen, versetzt angeordnet sind, wenn diese zweiten Greifbackenabschnitte unter der Wirkung von zwei entgegenwirkenden elastischen Kräften, die auf die ersten Greifbackenabschnitte (111, 112) ausgeübt werden, im Wesentlichen miteinander in Kontakt gelangen.

7. System nach einem der vorhergehenden Ansprüche, **dadurch gekennzeichnet, dass** die Mittel (20), um die Schenkel (11, 12) so zusammenzusetzen, dass sie relativ zueinander um die Drehachse (21) drehbar sind, durch mindestens ein Drehsystem (PA) gebildet sind, das ein Wellenlager (22) und eine zu dem Wellenlager komplementäre Welle (23) umfasst, wobei das Wellenlager durch einen im Wesentlichen rotationssymmetrischen zylindrischen Ring um die Drehachse (21) gebildet ist, derart, dass die Welle (23) in dem Ring einrasten kann.

8. System nach Anspruch 7, **dadurch gekennzeichnet, dass** es zwei im Wesentlichen gleiche Drehsysteme (PA) umfasst, wobei die zwei Drehsysteme voneinander entfernt sind, um zwischen sich einen freien Raum (Esl) freizugeben, in dem die beiden Schenkel (133, 134) angeordnet werden.

9. System nach Anspruch 8, wenn abhängig von Anspruch 5, **dadurch gekennzeichnet, dass** mindestens einer der zwei Schenkel (133, 134) des im Wesentlichen U-förmigen Teils (131) eine Breite (LB) besitzt, die im Wesentlichen gleich jener des freien Raums (Esl) ist.

10. System nach einem der vorhergehenden Ansprüche, wenn abhängig von Anspruch 3, **dadurch gekennzeichnet, dass** der Mittelteil (261) des Stifts (260) in einer Weise gebogen ist, dass er eine geschlossene Schleife bildet, und dass sich die zwei zweiten Teile (262, 263) außerhalb der Schleife befinden.

## Claims

1. A system for bringing two blood vessels (Vs) into abutment, the system comprising at least two clamps (Pc), each clamp comprising: two substantially identical tabs (11, 12), means (20) for pivotally mounting said two tabs (11, 12) relative to each other about a pivot axis (21), said pivot axis (21) being situated substantially in the proximity of respective middle portions (13, 14) of the two tabs, thereby defining on each of the tabs first and second portions (111, 112; 211, 212) situated on either side of a first plane (Pl) containing said pivot axis (21) and substantially perpendicular to the general plane of at least one of the two tabs; means (30) for applying two respective opposing resilient forces on the two first tab portions (111, 112) so that the two free ends (15, 16) of these two tab portions (111, 112) tend to move apart from each other and, correspondingly, the free ends (17, 18) of the two second tab portions (211, 212) tend to come into contact with each other; and a through hole (260) made in one of the two tabs along an axis substantially parallel to said pivot axis (21), said through hole being cylindrical and of polygonal cross-section; said system further including a rod (260) of longitudinal axis (270) and having a cross-section that is complementary to the cross-section of the through hole, said rod being engaged in the two respective through holes (250) of the two clamps, a portion of said rod extending between the two clamps possessing two moments of inertia of different values, **characterized by** the fact that the smaller value moment of inertia is the moment of inertia defined along the direction that is perpendicular to said first plane (Pl).

2. A system according to claim 1, **characterized by** the fact that said rod (260) is constituted by a plane plate defined over its entire length in a single plane and having thickness that is less than its width, and that the cross-section of the through hole (250-1) is substantially rectangular, said through hole presenting a depth defined in a direction parallel to said pivot axis (21), and a width and a length defined respectively in two directions that are perpendicular to said pivot axis (21), its length being substantially parallel to the first plane (Pl).

3. A system according to claim 1, **characterized by** the fact that said rod (260) is constituted by a plate having a middle first portion (261) defined in a plane and situated between two second portions (262, 263) defined in a plane substantially perpendicular to the plane of the first portion, and that the cross-section of the through hole (250-2) is substantially rectangular, said through hole presenting a depth defined in a direction parallel to said pivot axis (21), and a width and a length defined respectively along two directions perpendicular to said pivot axis (21), its length being substantially perpendicular to the first plane (PI), the two second portions (262, 263) of the rod being engaged respectively in the two respective through holes (250-2) of the two clamps.

4. A system according to any one of claims 1 to 3, **characterized by** the fact that said means (30) for applying two opposing resilient forces respectively against the two first portions of the tabs (111, 112), are constituted by a substantially U-shaped part (131) formed in a plate (132) made of an elastic material that is folded so as to have two branches (133, 134), one end of one of the two branches being connected to one end of the other branch by a half-loop (135) that is open over at least one hundred and eighty degrees of angle, said U-shaped part being engaged between the two tabs (11, 12), and that said clamp also includes means for connecting together in abutment the two free ends (136, 137) of the two respective branches (133, 134) respectively with the facing faces of the two first tab portions (111, 112).

5. A system according to claim 4, **characterized by** the fact that the means for bringing the two free ends (136, 137) of the two branches (133, 134) into abutment respectively against the facing faces of the two tab portions (111, 112) are constituted by recessed housings (141, 142) made in the facing faces of these first tab portions (111, 112) that receive the respective free ends (136, 137) of the two branches (133, 134) of said U-shaped part.

6. A system according to any preceding claim, **characterized by** the fact that each of the faces of the second tab portions (211, 212) that face each other includes a plurality of projecting studs (311, 312), said studs being arranged on said faces in such a manner that the studs situated on the faces of one of the second tab portions (211, 212) are arranged in staggered configuration relative to the studs situated on the face of the other second tab portion (212, 211) when these second tab portions come substantially into contact with each other under the action of the opposing resilient forces applied to the first tab portions (111, 112).

7. A system according to any preceding claim, **characterized by** the fact that said means (20) for mounting said branches (11, 12) to pivot relative to each other about said pivot axis (21) are constituted by at least one pivot system (PA) comprising a bearing (22) and a shaft (23) complementary to said bearing, said bearing being constituted by a cylindrical ring substantially in the form of a body of revolution around said pivot axis (21) so that said shaft (23) can be clipped in said ring.

8. A system according to claim 7, **characterized by** the fact that it includes two substantially identical pivot systems (PA), said two pivot systems being spaced apart from each other so as to leave an empty space (Esl) between them, in which said two branches (133, 134) take up position.

9. A system according to claim 8 when dependent on claim 5, the system being **characterized by** the fact that at least one of the two branches (133, 134) of said substantially U-shaped part (131) has a width (LB) that is substantially equal to the width of said empty space (Esl).

10. A system according to any preceding claim when dependent on claim 3, **characterized by** the fact that said middle portion (261) of the rod (260) is bent so as to form a closed loop and that said two second portions (262, 263) are situated outside said loop.
